# EUROPÄISCHE PATENTSCHRIFT

(11) **EP 1 509 194 B1**
(45) Veröffentlichungstag und Bekanntmachung des Hinweises auf die Patenterteilung: **04.08.2010**
(21) Anmeldenummer: 03740141.1
(22) Anmeldetag: 17.05.2003
(51) Int. Cl.: A61Q 17/04, A61K 8/04, A61K 8/27, A61K 8/29, A61K 8/55, A61K 8/81, B01F 17/00

(54) **HOCHKONZENTRIERTE WAESSRIGE DISPERSIONEN ENTHALTEND HYDROPHOBE MIKROFEINE METALLOXIDPARTIKEL UND DISPERGIERHILFSMITTEL**
HIGHLY CONCENTRATED AQUEOUS DISPERSIONS CONTAINING HYDROPHOBIC MICROFINE METAL OXIDE PARTICLES AND A DISPERSANT
DISPERSIONS AQUEUSES HAUTE CONCENTRATION RENFERMANT DES PARTICULES D'OXYDE METALLIQUE HYDROPHOBES MICROFINES ET UN AGENT DISPERSANT

(30) Priorität: 06.06.2002 DE 10225122
(43) Veröffentlichungstag der Anmeldung: 02.03.2005
(73) Patentinhaber: Evonik Goldschmidt GmbH, 45127 Essen (DE); Evonik Degussa GmbH, 45128 Essen (DE)
(72) Erfinder: BATZ-SOHN, Christoph, 63454 Hanau (DE); BRANDT, Petra, 47169 Duisburg (DE); DIETZ, Thomas, 45127 Essen (DE); HASENZAHL, Steffen, NJ, 07950 (US); JENNI, Klaus, 45357 Essen (DE); LEHMANN, Kathrin, 51377 Leverkusen (DE); MATHIAK, Ralf, 45968 Gladbeck (DE); RUETTGERODT, Angela, 51069 Köln (DE)
(86) Internationale Anmeldenummer: PCT/EP2003/005204
(87) Internationale Veröffentlichungsnummer: WO 2003/103620

(56) Entgegenhaltungen:
- EP-A- 0 559 320
- WO-A-00/49099
- WO-A-96/41614
- WO-A-97/15382
- US-A- 5 902 569
- US-A- 6 051 627
- "Römpp Chemie Lexikon" 1996, J.FALBE, M.REGITZ * Seite 1010 *

## Beschreibung

Die Erfindung betrifft wässrige Dispersionen enthaltend hydrophobe mikrofeine Metalloxidpartikel und als Dispergierhilfsmittel eine Kombination aus Phosphatestern und Maleinsäureanhydrid-Acrylat-Copolymeren und die Verwendung dieser Dispersionen zur Herstellung kosmetischer Formulierungen, insbesondere Sonnenschutzformulierungen.

Zum Schutz der Haut gegen zu intensive W-Strahlung werden UV-Filter enthaltende kosmetische Zubereitungen, wie Cremes oder Lotionen verwendet, die auf der Haut weitgehend transparent und angenehm in der Anwendung sind.

Als UV-Filter enthalten sie eine oder mehrere organische Verbindungen, die im Wellenlängenbereich zwischen 290 und 400 nm absorbieren: UVB- (290 bis 320 nm); WA-Strahlung (320 bis 400 nm).

Die energiereichere WB-Strahlung verursacht die typischen Sonnenbrandsymptome und ist auch verantwortlich für die Unterdrückung der Immunabwehr, während die tiefer in die Hautschichten eindringende WA-Strahlung die vorzeitige Alterung der Haut verursacht. Da das Zusammenwirken beider Strahlungsarten das Entstehen von lichtbedingten Hautkrebserkrankungen wie Hautkrebs begünstigen soll, begann daher frühzeitig die Suche nach Möglichkeiten, den bereits erzielten W-Schutz nochmals signifikant zu verbessern.

Es wurde gefunden, dass mikrofeine (ultrafeine) Pigmente auf Basis von Metalloxiden auch UV-Strahlung streuen, reflektieren und absorbieren können. Daher stellen ihre hochdispersen Formulierungen eine effektive Ergänzung der organischen UV-Filter in Sonnenschutzmitteln dar.

Mikrofeines Titandioxid wird in kosmetischen Formulierungen vielfältig verwendet, da es chemisch inert und toxikologisch unbedenklich ist und weder zu Hautirritationen noch zur Sensibilisierung führt. Es ist der derzeit am häufigsten verwendete und wichtigste mineralische Lichtschutzstoff. Neben Titandioxid wird in zunehmendem Maße mikrofeines Zinkoxid eingesetzt.

Es wird unterschieden zwischen grobteiligem Material (Pigment) und feinteiligem Material (Mikropigment). Bei den Mikropigmenten liegt die mittlere Primärteilchengröße in der Regel deutlich unter 200 nm, meist im Bereich von 10 bis 100 nm, in der Regel unter 50 nm.

Das grobteilige Pigment (0,2 bis 0,5 µm) absorbiert bzw. reflektiert breit und relativ gleichbleibend über den gesamten UV-Bereich und den Bereich des sichtbaren Lichtes während das feinteilige Material eine deutliche Wirkungserhöhung im UV-Bereich bei gleichzeitigem Wirkungsverlust im langwelligen UV-A und insbesondere im sichtbaren Bereich zeigt. Da nur noch wenig sichtbares Licht reflektiert wird, sind Präparate auf dieser Wirkstoffbasis daher weitgehend transparent.

Aufgrund ihrer besonders großen spezifischen Oberflächen sind die mikrofeinen TiO₂-Partikel photoaktiv und in der Lage, reaktive Spezies (z. B. Hydroxylradikale) zu generieren. Für den Einsatz in kosmetischen Mitteln ist es daher erforderlich, die photochemische Aktivität zu unterdrücken. Dies geschieht durch anorganische und organische Oberflächenkomponenten wie beispielsweise Al₂O₃, SiO₂ und/oder Fettsäure(salze), Siloxane. Diese Substanzen können chemisorptiv oder physisorptiv an der Oberfläche haften (Gitterdotierung/Coating). Dies erst führt zu Qualitäten, die sich für kosmetische Lichtschutzmittel eignen.

Die Primärpartikel des mikrofeinen Titandioxids liegen im trockenen Pigmentpulver nicht in isolierter Form vor, sondern bilden Aggregate und Agglomerate.

Als Primärpartikel bezeichnet man die kleinsten Teilchen, die bei der Herstellung der Pigmente anfallen. Primärteilchen können in Form einzelner Kristallite oder aber in Form mehrerer dicht über Flächen miteinander verwachsener Kristallite vorliegen. Als Aggregate bezeichnet man Teilchen, die sich aus mehreren Primärteilchen zusammensetzen, wobei die Primärteilchen flächig miteinander verwachsen sind. Unter einem Agglomerat versteht man einen Verband von Primärteilchen oder Aggregaten, die über anziehende Kräfte, wie z.B. Wasserstoffbrücken-Bindungen zusammengehalten werden.

Agglomerate sind in jedem Pigmentpulver enthalten, in kosmetischen transparenten Formulierungen jedoch unerwünscht, da sie als Partikel auf der Haut oft bereits mit dem bloßen Auge zu erkennen sind, die Transparenz als auch die W-Schutzwirkung eines Sonnenschutzmittels mindern und sich bei Lagerung absetzen. Sie müssen daher weitgehend wieder zerkleinert werden.

Den gesamten Prozess des Einbringens, Zerteilens und gleichmäßigen Verteilens von Feststoffen in einer flüssigen Phase bezeichnet man als Dispergieren.

Mit abnehmender Primärteilchengröße steigt die spezifische Oberfläche und damit die aktive Fläche zur Bildung von Aggregaten und Agglomeraten, sowie für Adsorptionsvorgänge, wodurch die Stabilität von Emulsionen gefährdet werden kann.

Das Zerteilen der Agglomerate und Benetzen der neugeschaffenen Oberflächen ist nur mit Hilfe hoher Scherkräfte möglich und geschieht in der Praxis in einer Vielzahl von verschiedenen Spezialmaschinen, wie insbesondere Dissolvern und Kugelmühlen.

In der Praxis hat sich gezeigt, dass mit wachsender Feinteiligkeit der Partikel die Dispergierprobleme zunehmen, so dass der Dispergierprozess insgesamt einen der aufwendigsten Teilschritte bei der Herstellung von kosmetischen Formulierungen darstellt.

Die Forderungen der Praxis gehen daher dahin, den aufwendigsten Teil der Dispergierung - die Zerteilung der Agglomerate - von der Herstellung der eigentlichen kosmetischen Formulierungen zu trennen und stabile wässrige Dispersionen mit möglichst hohem Gehalt an mikrofeinem TiO₂ bereitzustellen, welche vorzugsweise niedrigviskos oder zumindest noch pumpbar oder fließfähig sind.

Es wurde daher eine Vielzahl von Vorschlägen gemacht, die dieses Problem lösen sollten.

Die GB-A-2 206 339 beschreibt Dispersionen von Titandioxidpartikeln einer Partikelgröße von 0,01 bis 0,15 µ in organischen Ölen und Dispergierhilfsmitteln auf Basis von Polyestern, Salzen von Hydroxycarbonsäuren und/oder hydroxylgruppenfreien C₆₋₂₂-Fettsäuren oder deren Salzen und deren Verwendung als Sonnenschutzmittel.

So wird in der WO-A-90/06103 vorgeschlagen, die Verklumpungsneigung (Tendenz zur Reagglomeration von Titandioxidpartikeln mit Korngrößen < 100 nm) durch Überzüge aus Phospholipiden zu reduzieren.

Die DE-A-39 41 543 beschreibt ein Verfahren zur Herstellung wässriger Dispersionen von nadelförmigem feinteiligem Titandioxid, welches gegebenenfalls mit wasserhaltigen Metalloxiden überzogen ist, durch Vermahlen der Titandioxidpartikel in Gegenwart einer Polycarbonsäure oder deren Salz als Dispersionsmittel und die Verwendung als Sonnenschutzmittel.

Diese Dispersionen weisen zwar tendenzielle Verbesserungen auf, haben aber immer noch den Nachteil, dass die wässrigen Dispersionen nicht ausreichend hohe Gehalte an mikrofeinem TiO₂ enthalten, bei Lagerung sedimentieren und/oder die Photoaktivität noch zu hoch ist.

Ein weiterer wesentlicher Nachteil besteht darin, dass sie in dem für kosmetische Formulierungen besonders bevorzugten pH-Bereich von ca. 5 bis 7 (pH-Wert der Hautoberfläche) nicht stabil sind.

Aufgabe der vorliegenden Erfindung war es daher, die bestehenden Nachteile zu überwinden und stabile, hochkonzentrierte wässrige Dispersionen von mikrofeinen Metalloxidpartikeln, insbesondere mikrofeinem Titandioxid, mit vergleichsweise niedrigen Viskositäten herzustellen, welche auch im sauren physiologisch günstigen pH-Bereich stabil sind.

Die Aufgabe wird gelöst, durch Verwendung von hydrophob gecoateten mikrofeinen Metalloxidpartikeln und einer Kombination aus Phosphatestern und Maleinsäureanhydrid-Acrylat Copolymeren als Dispergierhifsmitteln.

Hydrophob gecoatetes Titandioxid weist neben einer geringen photokatalytischen Aktivität eine geringe Tendenz zur Reagglomeration auf, da insbesondere die Ausbildung von Wasserstoffbrücken über partiell vorhandene Ti-OH-Gruppen benachbarter TiO₂-Partikel nicht oder nur in geringem Maße möglich ist. Da hydrophob gecoatete Titandioxide in der Regel nicht ausreichend wasserbenetzbar sind, sind wässrige Dispersionen dieser Stoffe bislang nicht bekannt.

Ein Gegenstand der Erfindung sind daher im sauren physiologisch günstigen pH-Bereich stabile wässrige Dispersionen enthaltend
A) 20 bis 60 Gew.-% bezogen auf die Gesamtdispersion hydrophob gecoatete mikrofeine Metalloxidpartikel mit Primärpartikelgrößen von im Durchschnitt < 250 nm und als Dispergierhilfsmittel
B) mindestens eine der Verbindungen der allgemeinen Formel (I) 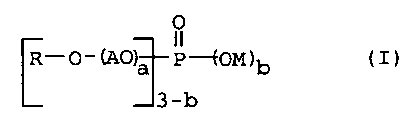 worin bedeuten
   - R: ein gegebenenfalls verzweigter, gegebenenfalls Mehr- fachbindungen, gegebenenfalls Hydroxylgruppen enthal- tender Akylrest mit 6 bis 22 C-Atomen,
   - A: Ethylenrest, Propylenrest, iso-Propylenrest, Butylen- rest,
   - M: H, Ammoniumion oder ein Alkalimetallkation
   - a: 0 bis 30,
   - b: 0 bis 2 und gegebenenfalls
C) mindestens eine der Verbindungen der allgemeinen Formel (II) 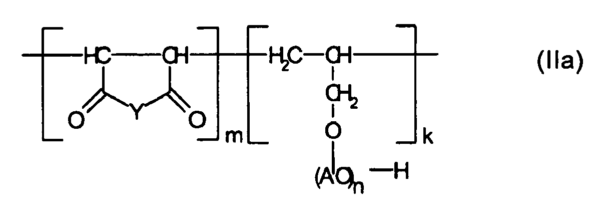 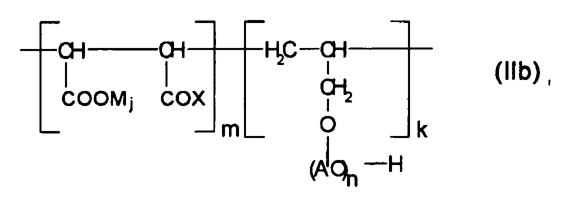 worin bedeuten
   - M: Wasserstoff, ein- oder zweiwertiges Metallkation, Ammoniumion, organischer Aminrest
   - j: 1, oder für den Fall, dass M ein zweiwertiges Metallkation ist, = 0,5
   - X: ebenfalls -OMⱼ oder -o-(CₚH₂ₚO)_{q} - R¹ mit R¹ = H, aliphatischer Kohlenwasserstoffrest mit 1 - 20 C Atomen, cycloali- phatischer Kohlenwasserstoffrest mit 5 bis 8 C-Atomen, ggf. substituierter Arylrest mit 6 bis 14 C-Atomen, p = 2 bis 4, q = 0 bis 100, -NHR² und/oder -NR²₂ mit R² = R¹ oder -CO-NH₂
   - Y: O, NR²
   - A¹: Ethylenrest, Propylenrest, iso-Propylenrest, Butylenrest,
   - m: 10 bis 30,
   - n: 0 bis 50,
   - k: 10 bis 30, wobei die Summe
   - m + k: im Bereich von 20 bis 60, vorzugsweise von 20 bis 40 liegt, gegebenenfalls
D) weitere Hilfs- und Zusatzstoffe
E) Wasser.

Ein weiterer Gegenstand der Erfindung ist die Verwendung der wässrigen Dispersionen gemäss Anspruch 1 zur Herstellung von kosmetischen Formulierungen.

Weitere Gegenstände der Erfindung sind durch die Ansprüche gekennzeichnet.

Die erfindungsgemäß verwendeten Phosphorester werden durch die allgemeine Formel (I) 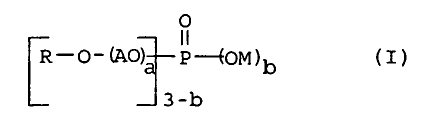 idealisiert dargestellt.

Bedingt durch das technische Herstellungsverfahren liegen Mischungen vor, in denen die gewünschte Hauptkomponente, erfindungsgemäß vorzugsweise der Monoester und der Diester, überwiegend neben geringen Anteilen der weiteren möglichen Reaktionsprodukte vorhanden sind.

Sie werden hergestellt durch Umsetzung von Fettalkoholen R-OH oder Fettalkoholalkoxilaten R-O-(AO)ₐ-H mit Phosphorsäure oder deren Derivaten nach bekannten Verfahren.

Die mitverwendeten Fettalkohole können nach bekannten Verfahren durch Reduktion von Fettsäuren oder deren Estern in Gegenwart von Katalysatoren hergestellt werden. Bei der Direkthydrierung werden Fettalkohole aus Triglyceriden in einem einstufigen Prozess in einem Rohrreaktor mit Wasserstoff an einem Cu-/Cr-Katalysator umgesetzt, wobei als Reaktionsprodukte Fettalkohol, 1,2-Propandiol u. Wasser entstehen. Bei anderen Verfahren wird Fettalkohol aus Triglyceriden über einen Umesterungsschritt mit anschließender Hydrierung des Fettsäureesters hergestellt.

Als Fettsäuren werden einzeln oder in Mischungen Fettsäuren wie Caprylsäure, Caprinsäure, 2-Ethylhexansäure, Laurinsäure, Myristinsäure, Palmitinsäure, Palmitoleinsäure, Isostearinsäure, Stearinsäure, Hydroxystearinsäure (Ricinolsäure), Dihydroxystearinsäure, Ölsäure, Linolsäure, Petroselinsäure, Elaidinsäure, Arachinsäure, Behensäure und Erucasäure, Gadoleinsäure sowie die bei der Druckspaltung natürlicher Fette und Öle anfallenden technischen Mischungen wie Ölsäure, Linolsäure, Linolensäure, und insbesondere Rapsölfettsäure, Sojaölfettsäure, Sonnenblumenölfettsäure, Tallölfettsäure mitverwendet. Geeignet sind prinzipiell alle Fettsäuren mit ähnlicher Kettenverteilung.

Der Gehalt dieser Fettsäuren bzw. Fettsäureester an ungesättigten Anteilen, wird - soweit dies erforderlich ist - durch die bekannten katalytischen Hydrierverfahren auf eine gewünschte Jodzahl eingestellt oder durch Abmischung von vollhydrierten mit nichthydrierten Fettkomponenten erzielt. Die Jodzahl, als Maßzahl für den durchschnittlichen Sättigungsgrad einer Fettsäure, ist die Jodmenge, welche von 100 g der Verbindung zur Absättigung der Doppelbindungen aufgenommen wird.

Vorzugsweise werden die Alkohole aus teilgehärteten C_{8/18}-Kokos- bzw. Palmfettsäuren, Rapsölfettsäuren, Sonnenblumenölfettsäuren Sojaölfettsäuren und Tallölfettsäuren, mit Jodzahlen im Bereich von ca. 80 bis 150 und insbesondere aus technischen C_{8/18}-Kokosfettsäuren eingesetzt, wobei gegebenenfalls eine Auswahl von cis-/trans-Isomeren wie elaidinsäurereiche C_{16/18}-Fettsäureschnitte von Vorteil sein können. Sie sind handelsübliche Produkte und werden von verschiedenen Firmen unter deren jeweiligen Handelsnamen angeboten.

Neben den Fettalkoholen können insbesondere auch Guerbetalkohole und deren Alkoxilate mitverwendet werden.

Die Alkoholalkoxilate R-O-(AO)ₐ-H können nach den bekannten Verfahren durch Anlagerung von Alkylenoxiden in Gegenwart von sauren oder basischen Katalysatoren erhalten werden.
Der Rest -(AO)ₐ- steht hier für Reste wie Ethylenoxid, Propylenoxid, Butylenoxid und/oder Tetrahydrofuran, vorzugsweise Ethylenoxid, wobei a für einen Durchschnittswert von bis zu 30, vorzugsweise 3 bis 15 Einheiten steht.

In der allgemeinen Formel bedeutet -(AO)ₐ- sowohl ein Homopolymerisat aus einer der genannten Alkylenoxide als auch Block-Copolymerisate oder Co-Polymerisate mit statistischer Verteilung von zwei oder mehreren der Monomeren im Polymermolekül.

Diese Produkte sind handelsüblich. Sie werden in Mengen von 0,5 bis 30 % bezogen auf wässrige Dispersionen, vorzugsweise von 3 bis 15 % bezogen auf wässrige Dispersionen mitverwendet.

In den erfindungsgemäß gegebenenfalls mitverwendeten Verbindungen der allgemeinen Formel II 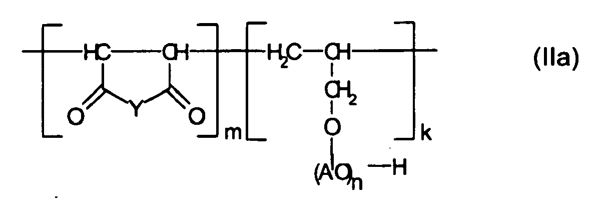 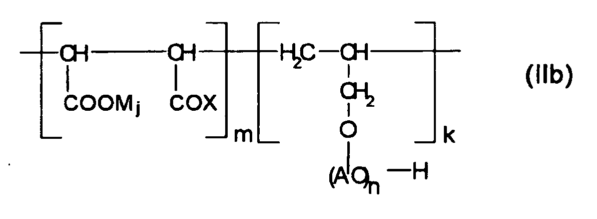 bedeuten
- M: Wasserstoff, ein- oder zweiwertiges Metallkation, Ammoniumion, organischer Aminrest
- j: 1, oder für den Fall, dass M ein zweiwertiges Metallkation ist, =0,5
- X: ebenfalls -OMⱼ oder -O-(CₚH₂ₚO)_{q} - R¹ mit R¹ = H, aliphatischer Kohlenwasserstoffrest mit 1 - 20 C Atomen, cycloali- phatischer Kohlenwasserstoffrest mit 5 bis 8 C-Atomen, ggf. substituierter Arylrest mit 6 bis 14 C-Atomen, p = 2 bis 4, q = 0 bis 100, -NHR² und/oder -NR²₂ mit R² = R¹ oder -CO-NH₂
- Y: O, NR²
- A¹: Ethylenrest, Propylenrest, iso-Propylenrest, Butylenrest,
- m: 10 bis 30,
- n: 0 bis 50,
- k: 10 bis 30, wobei die Summe
- m + k: im Bereich von 20 bis 60, vorzugsweise von 20 bis 40 liegt.
-(AO)ₙ- sowohl ein Homopolymerisat aus einer der genannten Alkylenoxide als auch Block-Copolymerisate oder Co-Polymerisate mit statistischer Verteilung von zwei oder mehreren der Monomeren im Polymermolekül,
die Einheiten
[ ]ₘ und [ ]ₖ können ebenfalls als Block-Copolymerisate oder Co-Polymerisate mit statistischer Verteilung von zwei oder mehreren der Monomeren im Polymermolekül vorliegen.

Diese Produkte werden in Mengen bis 30 Gew.-%, vorzugsweise in Mengen von 1 bis 15 Gew.-%, bezogen auf wässrige Dispersionen mitverwendet.

Als mikrofeine Metalloxidpartikel können grundsätzlich alle in den jeweiligen Anwendungsgebieten üblichen Metalloxide verwendet werden. Die Bezeichnung "mikrofein" oder "ultrafein" bezieht sich dabei auf Primärpartikelgrößen von im Durchschnitt < ca. 250 nm, vorzugsweise auf ca. 100 nm und darunter. Für die Verwendung in kosmetischen Formulierungen beschränkt sich die Auswahl naturgemäß auf die aus toxikologischer und dermatologischer Sicht unbedenklichen Verbindungen wie vorzugsweise Ceroxid, Zinkoxid, Eisenoxid und insbesondere Titandioxid.

Die erfindungsgemäß mitverwendeten mikrofeinen Metalloxidpartikel sind handelsübliche Produkte, die unter den jeweiligen Markennamen, auch mit anorganischen oder organischen Beschichtungen, erhältlich sind, wie beispielsweise Micro Titanium Dioxide MT-500B, MT-100 Z und MT-100 TV (alle Tri-K-Tayca), UV-Titan M 160, M 262, X 161 (alle Kemira), Eusolex T-2000 (Merck), Uvinul TiO₂ (BASF), Titandioxid T-805 und Titandioxid T-817 (beide Degussa).

Erfindungsgemäß bevorzugt wird Titandioxid mit mittleren Primärpartikelgrößen von < 100 nm und insbesondere im Bereich von 10 bis 100, welche mit Fettsäure(salzen) und insbesondere Alkylsilanen überzogen sind.

Als besonders vorteilhaft hat sich hier das Titandioxid T 805 (Degussa) erwiesen. Titandioxid T 805 besteht kristallographisch aus etwa 80 % Anatas und etwa 20 % Rutil und hat eine mittlere Primärpartikelgröße von etwa 21 nm und ist mit Trialkoxyoctylsilan gecoatet. Es zeichnet sich durch verminderte Photoaktivität, verringerte Oberflächenaktivität, hohe kosmetische Akzeptanz, sehr gute Wasserfestigkeit aus.

Zusätzlich zu den genannten Komponenten können bei Bedarf weitere auf diesem Gebiet bekannte Hilfs- und Zusatzstoffe mitverwendet werden wie Ethanol, Propanol, Butanol, Propylenglycol, Butylenglycol, Pentylenglycol, Hexylenglycol, Alkoxilate, Glykolether, Glykole, Polyethylenglykole, Polypropylenglykole, Polybutylenglykole, Glycerinesterethoxylate, Glycerin, Polyglycerin, Sorbitol, Sucrose, Fructose, Galactose, Mannose, Polysorbate, Stärke, Xanthan Gum, Carrageenan Gum, CelluloseDerivate, Alginate, Glycol Ester, Sorbitanester, Trübungsmittel, Solubilisatoren, ethoxylierte Fettalkohole, Natriumchlorid, Natriumsulfat, Magnesiumsulfat, Puffer-Systeme, Cholesterin, Panthothensäure, Ascorbinsäure, Polyacrylsäuren, Carbomere.

Die erfindungsgemäßen Dispersionen werden vorzugsweise zur Herstellung kosmetischer Formulierungen wie Make-up, colorierte Puder, Lippenstifte, Haarcolorierungsmittel, Tagescremes und insbesondere Sonnenschutzpräparaten verwendet und können in den üblichen Formen wie beispielsweise als W/O- oder O/W-Dispersionen (Emulsionen), Gele, Cremes, Lotionen, Sprays vorliegen.

Die erhaltenen Dispersionen zeichnen sich durch hohe Feinteiligkeit des dispergierten Festsoffes, langfristige Lagerstabilität, niedrige Viskosität und hohe Photostabilität aus.

Die Viskosität wird gemessen mit einem Brookfield RVT, Spindel 5, nach den Angaben des Herstellers und liegt bei Raumtemperatur bei 10 Umdrehungen pro Minute (Upm) zwischen 10 und 40.000 mPas.

Als ein Merkmal für die elektrostatische Stabilisierung einer Dispersion kann das Zetapotential herangezogen werden.

Das Zetapotential ist das nach außen wirksame Potential der Teilchen und stellt ein Maß der elektrostatischen Wechselwirkung zwischen einzelnen Partikeln dar. Es spielt eine Rolle bei der Stabilisierung von Suspensionen und insbesondere von Dispersionen mit dispergierten mikrofeinen Partikeln. Bei einem Zetapotentialwert von < als -20 mV oder > +20 mV besteht eine starke Abstoßung zwischen den Partikeln, die Dispersionen bleiben stabil. Bei Werten innerhalb dieses Bereichs wird die Abstoßung so gering, dass die van-der-Waal'schen Kräfte die Bildung von Agglomeraten erlauben und es zur unerwünschten Sedimentation der Partikel führt.

Die erfindungsgemäßen Dispersionen sind nach den auf diesem Gebiet allgemein bekannten Verfahren herstellbar, wobei als Mischgeräte Dispermaten mit Zahnscheiben, Perlmühlen, Rotor-Stator-Systeme, oder der Scandex-Schüttler verwendet werden.

Zweckmäßiger Weise werden die Dispergieradditive und ggf. mitverwendete Polyole in dem Wasser vorgelegt und das Pigment unter angemessenem Rühren eingestreut. Die so erhaltene Predispersion wird anschließend fein dispergiert.

Die wässrigen Dispersionen enthalten:
20 bis 60 Gew.-% der Komponente A
0,5 bis 30 Gew.-% der Komponente B), insbesondere 3 bis 15 %,
0 bis 30 Gew.-% der Komponente C), insbesondere 1 bis 15 %,
0 bis 20 Gew.-% der Komponente D), insbesondere 1 bis 10 %,
ad 100 Gew.-% Wasser.

Als Hilfs- und Zusatzstoffe können mitverwendet werden Glycerin, Propylenglycol, Butylenglykol und höhere Glykole, Polyglycerine, Sorbitol und vergleichbare Zuckeralkohole sowie 0,1 bis 0,5 % wasserlösliche bzw. wasserdispergierbare Konservierungsmittel.

### Formulierungsbeispiele:

### Beispiel 1 bis 4:

| Beispiel | 1 | 2 | 3 | 4 |
|---|---|---|---|---|
| TiO₂ (Degussa T 805) | 40.0 % | 40.0 % | 40.0 % | 40.0 % |
| Verbindung der Formel II (MW 15.000) | 7.0 % | 6.0 % | 5.0 % | 8.0 % |
| Rewophat^{®} EAK 8190 Verbindung der Formel I | 5.0 % | 6.0 % | 7.0 % | - |
| Guerbetalkohol C12 EO-4-Phosphat Formel I, b = 1 bis 2; M = H | - | - | - | 6.0 % |
| Glycerin | 10.0 % | 10.0 % | 10.0 % | 10.0 % |
| Wasser | 38.0 % | 38.0 % | 38.0 % | 36.0 % |
| Viskosität (mPas) | 340 | 477 | 430 | 1.400 |

### Beispiel 5 bis 9:

| Beispiel | 5 | 6 | 7 | 8 |
|---|---|---|---|---|
| UV-Titan M 160⁽¹⁾ | 40.0 % | | | |
| UV-Titan M 262 ⁽²⁾ | - | 40 % | 40 % | 40 % |
| Verbindung der Formel II (MW 15.000) | 0.5 % | 11.8 % | 0.5 % | - |
| Guerbetalkohol C18 EO-4-Phosphat Formel I, b = 1 bis 2; M = H | 15.5 % | 0.5 % | 11.5 % | 14 % |
| Glycerin | 10.0 % | 10.0 % | 10.0 % | 10 % |
| Wasser | 34.0 % | 37.7 % | 38.0 % | 36 % |
| Viskosität (mPas) | 1.200 | 600 | 100 | 360 |

| | | | | |
|---|---|---|---|---|
| ⁽¹⁾ Handelsprodukt der Firma Kemira, Finnland; mit Stearinsäure gecoatet. ⁽²⁾ Handelsprodukt der Firma Kemira, Finnland; mit Dimethicone gecoatet. | | | | |

### Beispiel 9:

| | |
|---|---|
| Beispiel | 9 |
| TiO₂ (Degussa T 805) | 40.0 % |
| Verbindung der Formel II (MW 15.000) | 3.0 % |
| Guerbetalkohol C18-4, EO-4-Phosphat Formel I, b = 1 bis 2; M = H | 9.0 % |
| Glycerin | 10.0 % |
| Wasser | 38.0 % |
| Viskosität (mPas) | 2.800 |

### Bestimmung des Zeta-Potentials:

Die Messungen wurden mit dem Gerät DT-1200 der Firma Dispersion Technology, USA nach der CVI-Methode (collodial vibration current) durchgeführt.

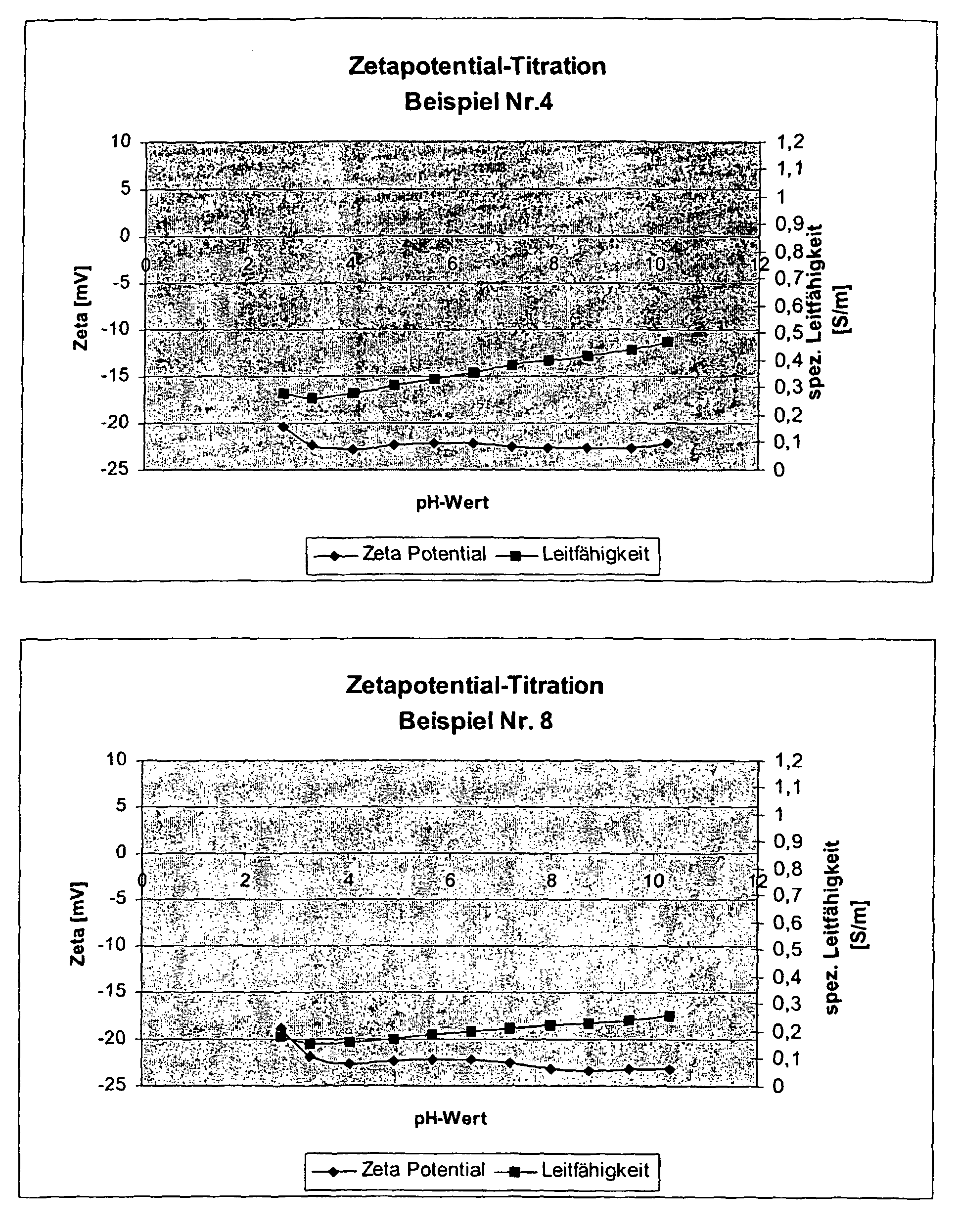

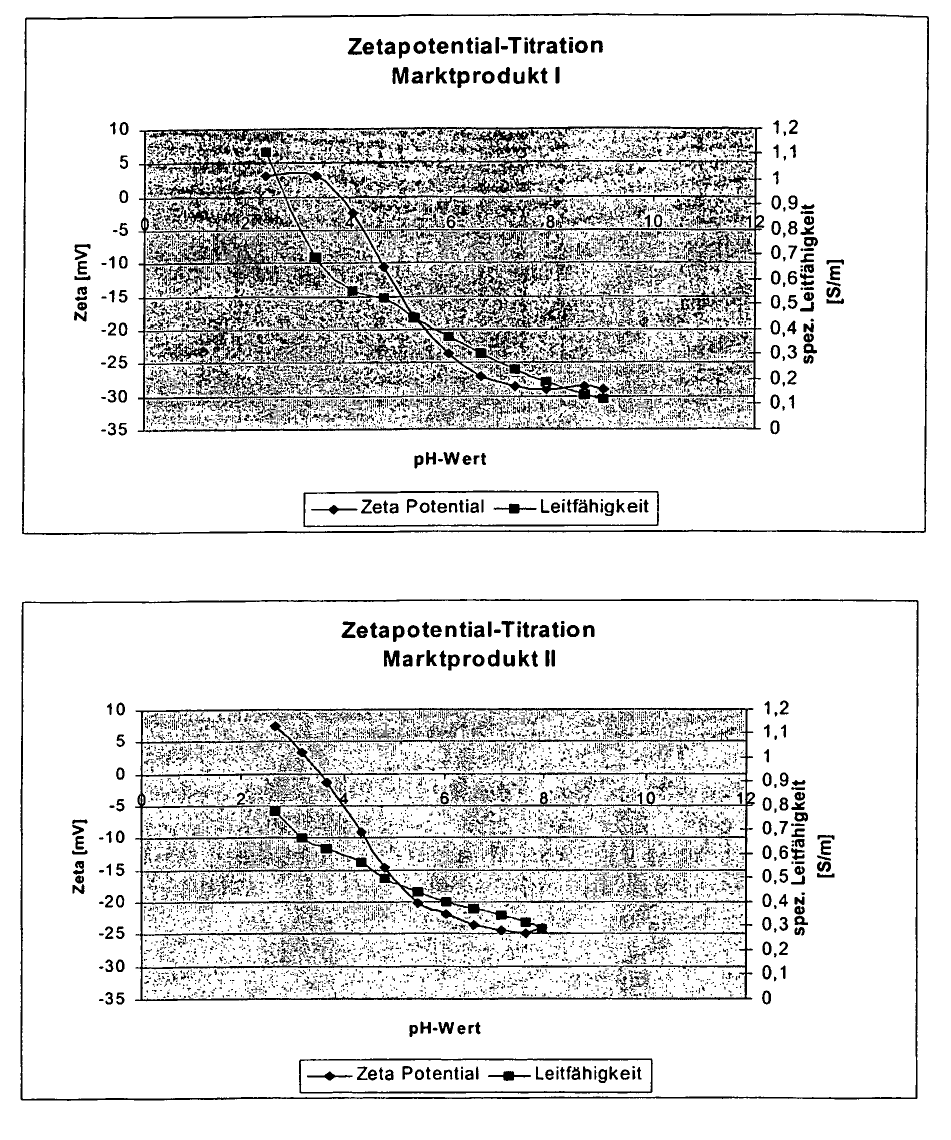

Aus den Kurven ist ersichtlich, dass das Zeta-Potential der erfindungsgemäßen Dispersionen im gesamten für kosmetische Formulierungen relevanten pH-Bereich und auch darüber hinaus < -20 mV beträgt, d.h. die Dispersionen ausgeprägt stabil sind.

Demgegenüber weisen handelsübliche, für kosmetische Formulierungen verwendete TiO₂-Dispersionen nur in einem relativ engen pH-Bereich ein ausreichendes Zeta-Potential auf und erreichen im kosmetisch interessanten pH-Bereich, um ca. 3 bis 5, den isoelektrischen Punkt.

## Patentansprüche

1. Im sauren physiologisch günstigen pH-Bereich stabile, wässrige Dispersionen enthaltend:
A) 20 bis 60 Gew.-% bezogen auf die Gesamtdispersion hydrophob gecoatete mikrofeine Metalloxidpartikel mit Primärpartikelgrößen von im Durchschnitt < 250 nm und
B) mindestens eine der Verbindungen der allgemeinen Formel (I) 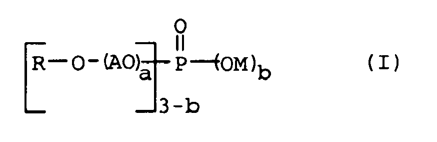 worin bedeuten
R ein gegebenenfalls verzweigter, gegebenenfalls Mehrfachbindungen, gegebenenfalls Hydroxylgruppen enthaltender Alkylrest mit 6 bis 22 C-Atomen,
A Ethylenrest, Propylenrest, iso-Propylenrest, Buty- lenrest,
M H, Ammoniumion oder ein Alkalimetallkation
a 0 bis 30,
b 0 bis 2 und gegebenenfalls
C) mindestens eine der Verbindungen der allgemeinen Formel (II) 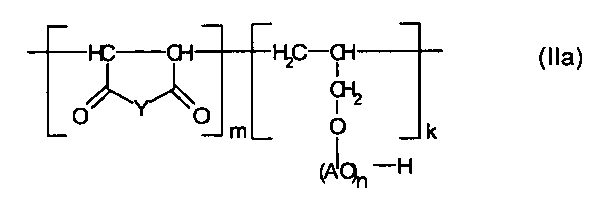 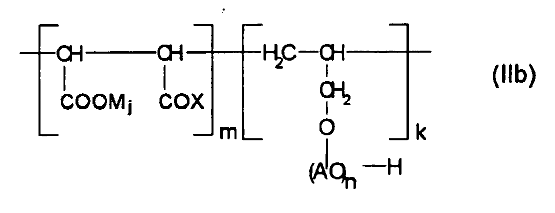 worin bedeuten
M Wasserstoff, ein- oder zweiwertiges Metallkation, Ammoniumion, organischer Aminrest
j 1, oder für den Fall, dass M ein zweiwertiges Metallkation ist, = 0,5
X ebenfalls -OMⱼ oder -O-(CₚH₂ₚO)_{q} - R¹ mit R¹ = H, aliphatischer Kohlenwasserstoffrest mit 1 - 20 C Atomen, cycloaliphatischer Kohlenwasserstoffrest mit 5 bis 8 C-Atomen, ggf. substituierter Arylrest mit 6 bis 14 C-Atomen, p = 2 bis 4, q = 0 bis 100, -NHR² und/oder -NR²₂ mit R² = R¹ oder -CO-NH₂
Y O, NR²
A¹ Ethylenrest, Propylenrest, iso-Propylenrest, Butylenrest,
m 10 bis 30,
n 0 bis 50,
k 10 bis 30, wobei die Summe
m + k im Bereich von 20 bis 60, vorzugsweise von 20 bis 40 liegt,
gegebenenfalls
D) weitere Hilfs- und Zusatzstoffe
E) Wasser.

2. Wässrige Dispersionen gemäss Anspruch 1, **dadurch gekennzeichnet, dass** R der Alkylrest eines Fettalkoholes mit 6 bis 22 C-Atomen, vorzugsweise von 12 bis 18 C-Atomen ist und a einen Wert zwischen 1 und 30, vorzugsweise von 3 bis 15 bedeutet.

3. Wässrige Dispersionen gemäss Anspruch 1, **dadurch gekennzeichnet, dass** R der Alkylrest eines Guerbetalkoholes mit 6 bis 22 C-Atomen, vorzugsweise von 12 bis 18 C-Atomen ist und a einen Wert zwischen 1 und 30, vorzugsweise von 3 bis 15 bedeutet.

4. Wässrige Dispersionen gemäss den Ansprüchen 1 bis 3, **dadurch gekennzeichnet, dass** als mikrofeine Metalloxidpartikel mit Trialkoxyoctylsilan hydrophob gecoatetes Zinkoxid und/oder Titandioxid mitverwendet wird.

5. Wässrige Dispersionen gemäss den Ansprüchen 1 bis 4, **dadurch gekennzeichnet, dass** die Primärpartikelgrößen der Metalloxide zwischen 10 und 100 nm liegen.

6. Wässrige Dispersionen gemäß den Ansprüchen 1 bis 5, **dadurch gekennzeichnet, dass** als Komponente C) Verbindungen der allgemeinen Formel II mitverwendet werden, worin A¹ ein Ethylenrest ist, m 10 bis 30, n 5 bis 20, k 10 bis 30 und wobei die Summe m + k im Bereich von 20 bis 40 liegt.

7. Wässrige Dispersionen gemäß den Ansprüchen 1 bis 6, **dadurch gekennzeichnet, dass** als Komponente B) Verbindungen der allgemeinen Formel I mitverwendet werden, worin R ein gegebenenfalls verzweigter, gegebenenfalls Mehrfachbindungen, gegebenenfalls Hydroxylgruppen enthaltender Akylrest mit 8 bis 18 C-Atomen, A ein Ethylenrest, M = H oder ein Alkalimetall, a 1 bis 30, b 1 oder 2, bedeuten.

8. Wässrige Dispersionen gemäss den Ansprüchen 1 bis 7, **dadurch gekennzeichnet, dass** die Viskositäten, gemessen mit einem Brookfield RVT, Spindel 5, 10 Upm, zwischen 10 und 40.000 mPas liegen.

9. Verwendung der wässrigen Dispersionen gemäss Anspruch 1 bis 8 zur Herstellung von kosmetischen Formulierungen.

## Claims

1. Aqueous dispersions stable in the acidic physiologically favorable pH range and comprising:
A) 20 to 60% by weight, based on the total dispersion, of hydrophobically coated microfine metal oxide particles having primary particle sizes of an average < 250 nm and
B) at least one of the compounds of the general formula (I) 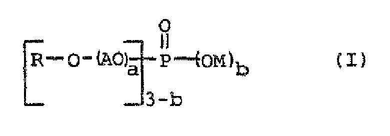 in which
R is an optionally branched alkyl radical having 6 to 22 carbon atoms which may or may not contain multiple bonds and may or may not contain hydroxyl groups,
A is ethylene radical, propylene radical, isopropylene radical, butylene radical,
M is H, ammonium ion or an alkali metal cation
a is 0 to 30,
b is 0 to 2 and optionally
C) at least one of the compounds of the general formula (II) 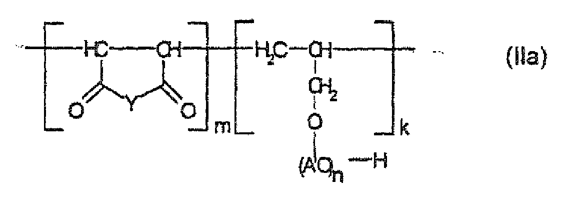 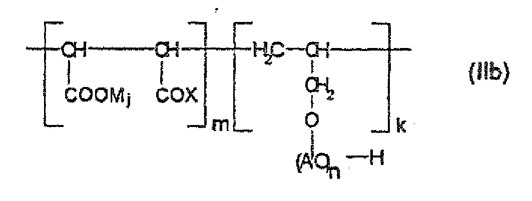 in which
M is hydrogen, monovalent or divalent metal cation, ammonium ion, organic amine radical
j is 1, or where M is a divalent metal cation, is 0.5
X is likewise -OMⱼ or -O- (CₚH₂ₚO)_{q}-R¹ where R¹ = H, aliphatic hydrocarbon radical having 1-20 carbon atoms, cycloaliphatic hydrocarbon radical having 5 to 8 carbon atoms, optionally substituted aryl radical having 6 to 14 carbon atoms, p = 2 to 4, q = 0 to 100, -NHR² and/or -NR²₂ where R² = R¹ or -CO-NH₂
Y is O, NR²
A¹ is ethylene radical, propylene radical, isopropylene radical, butylene radical,
m is 10 to 30,
n is 0 to 50,
k is 10 to 30, where the sum
m + k is in the range from 20 to 60, preferably from 20 to 40,
optionally
D) further auxiliaries and additives
E) water.

2. Aqueous dispersions according to Claim 1, **characterized in that** R is the alkyl radical of a fatty alcohol having 6 to 22 carbon atoms, preferably from 12 to 18 carbon atoms, and a is a value between 1 and 30, preferably from 3 to 15.

3. Aqueous dispersions according to Claim 1, **characterized in that** R is the alkyl radical of a Guerbet alcohol having 6 to 22 carbon atoms, preferably from 12 to 18 carbon atoms, and a is a value between 1 and 30, preferably from 3 to 15.

4. Aqueous dispersions according to Claims 1 to 3, **characterized in that** zinc oxide and/or titanium dioxide hydrophobically coated with trialkoxyoctylsilane is co-used as microfine metal oxide particles.

5. Aqueous dispersions according to Claims 1 to 4, **characterized in that** the primary particle sizes of the metal oxides are between 10 and 100 nm.

6. Aqueous dispersions according to Claims 1 to 5, **characterized in that** compounds of the general formula II in which A¹ is an ethylene radical, m is 10 to 30, n is 5 to 20, k is 10 to 30 and where the sum m + k is in the range from 20 to 40, are co-used as component C).

7. Aqueous dispersions according to Claims 1 to 6, **characterized in that** compounds of the general formula I in which R is an optionally branched alkyl radical having 8 to 18 carbon atoms which may or may not contain multiple bonds and may or may not contain hydroxyl groups, A is an ethylene radical, M = H or an alkali metal, a is 1 to 30, b is 1 or 2, are co-used as component B).

8. Aqueous dispersions according to Claims 1 to 7, **characterized in that** the viscosities, measured using a Brookfield RVT, spindle 5, 10 rpm, are between 10 and 40 000 mPas.

9. Use of the aqueous dispersions according to Claims 1 to 8 for the preparation of cosmetic formulations.

## Revendications

1. Dispersions aqueuses stables dans l'intervalle de pH physiologiquement favorable, contenant :
A) de 20 à 60 % en poids, par rapport à la dispersion totale, de particules microfines d'oxyde métallique à enrobage hydrophobe, ayant des tailles de particule primaire en moyenne < 250 nm et
B) au moins un des composés de formule générale (I) 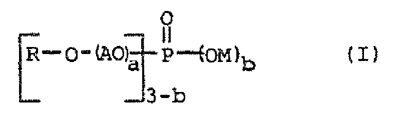 dans laquelle
R représente un radical alkyle ayant de 6 à 22 atomes de carbone, éventuellement ramifié, contenant éventuellement des liaisons multiples, éventuellement des groupes hydroxy,
A représente un radical éthylène, un radical propylène, un radical isopropylène, un radical butylène,
M représente H, l'ion ammonium ou un cation de métal alcalin,
a vaut de 0 à 30,
b vaut de 0 à 2 et éventuellement
C) au moins un des composés de formule générale (II) 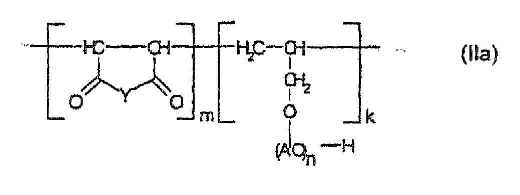 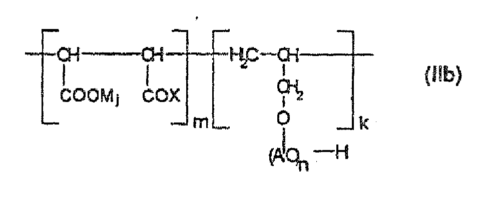 où
M représente un atome d'hydrogène, un cation métallique mono- ou divalent, l'ion ammonium, un radical amino organique
j vaut 1 ou, dans le cas où M est un cation métallique divalent, = 0,5
X représente aussi bien -OMⱼ que -O-(CₚH₂ₚO)_{q}-R¹, où R¹ = H, un radical hydrocarboné aliphatique ayant de 1 à 20 atomes de carbone, un radical hydrocarboné cycloaliphatique ayant de 5 à 8 atomes de carbone, un radical aryle éventuellement substitué ayant de 6 à 14 atomes de carbone, p = 2 à 4, q = 0 à 100, -NHR² et/ou -NR²₂, où R² = R¹ ou -CO-NH₂
Y représente O, NR²
A1 représente un radical éthylène, un radical propylène, un radical isopropylène, un radical butylène,
m vaut de 10 à 30,
n vaut de 0 à 50,
k vaut de 10 à 30,
la somme m + k se situant dans la plage allant de 20 à 60, de préférence de 20 à 40,
éventuellement
D) d'autres adjuvants et additifs
E) de l'eau.

2. Dispersions aqueuses selon la revendication 1, **caractérisées en ce que** R est le reste alkyle d'un alcool gras ayant de 6 à 22 atomes de carbone, de préférence de 12 à 18 atomes de carbone et a représente une valeur comprise entre 1 et 30, de préférence de 3 à 15.

3. Dispersions aqueuses selon la revendication 1, **caractérisées en ce que** R est le reste alkyle d'un alcool de Guerbet ayant de 6 à 22 atomes de carbone, de préférence de 12 à 18 atomes de carbone et a représente une valeur comprise entre 1 et 30, de préférence de 3 à 15.

4. Dispersions aqueuses selon les revendications 1 à 3, **caractérisées en ce qu'**on utilise en même temps comme particules microfines d'oxyde métallique du dioxyde de titane et/ou de l'oxyde de zinc à enrobage hydrophobe avec un trialcoxyoctylsilane.

5. Dispersions aqueuses selon les revendications 1 à 4, **caractérisées en ce que** les tailles de particule primaire des oxydes métalliques sont comprises entre 10 et 100 nm.

6. Dispersions aqueuses selon les revendications 1 à 5, **caractérisées en ce qu'**on utilise en même temps comme composant C) des composés de formule générale II, dans laquelle A¹ est un radical éthylène, m vaut de 10 à 30, n vaut de 5 à 20, k vaut de 10 à 30, la somme m + k se situant dans la plage allant de 20 à 40.

7. Dispersions aqueuses selon les revendications 1 à 6, **caractérisées en ce qu'**on utilise en même temps comme composant B) des composés de formule générale I, dans laquelle R représente un radical alkyle ayant de 8 à 18 atomes de carbone, éventuellement ramifié, contenant éventuellement des liaisons multiples, éventuellement des groupes hydroxy, A représente un radical éthylène, M = H ou un métal alcalin, a vaut de 1 à 30, b vaut 1 ou 2.

8. Dispersions aqueuses selon les revendications 1 à 7, **caractérisées en ce que** les viscosités, mesurées à l'aide d'un viscosimètre Brookfield RVT, broche 5, 10 tours/min, sont comprises entre 10 et 40 000 mPa.s.

9. Utilisation des dispersions aqueuses selon l'une quelconque des revendications 1 à 8, pour la préparation de compositions cosmétiques.
